Europäisches Patentamt

European Patent Office

Office européen des brevets

(11) Publication number: **0 263 731**
**A1**

# EUROPEAN PATENT APPLICATION

(21) Application number: 87402008.4

(51) Int. Cl.⁴: **G 01 N 33/546**

(22) Date of filing: 08.09.87

(30) Priority: 08.09.86 JP 210913/86

(43) Date of publication of application:
13.04.88 Bulletin 88/15

(84) Designated Contracting States: DE FR GB

(71) Applicant: MITSUBISHI CHEMICAL INDUSTRIES LIMITED
5-2, Marunouchi 2-chome Chiyoda-ku
Tokyo 100 (JP)

(72) Inventor: Minoru, Ogura
16-18 Sasage 2-chome Konan-ku
Yokohama-shi Kanagawa-ken (JP)

Kuninori, Sato
5-6 Tsutsujigaoka Midori-ku
Yokohama-shi Kanagawa-ken (JP)

(74) Representative: Peaucelle, Chantal et ai
S.C. Ernest Gutmann - Yves Plasseraud 67, boulevard Haussmann
F-75008 Paris (FR)

(54) Method for measuring antigen-antibody reactions.

(57) A latex reagent for measuring antigen-antibody reactions comprising at least two different kinds of latex having different average particle sizes. The reagent makes possible assay of antigen or antibodies with high sensitivity in a wide range of from low concentration to high concentration.

EP 0 263 731 A1

## Description

## Method for measuring antigen-antibody reactions

### Field of the Invention

This invention relates to a method of measuring antigen-antibody reactions. More particularly, this invention relates to a method of measuring antigen-antibody reactions comprising reacting latex particles which have been sensitized with an antibody or an antigen with an antigen against the antibody with which the latex has been sensitized or an antibody against the antigen with which the latex has been sensitized in an aqueous medium, irradiating the reaction mixture with light of a particular wavelength, and measuring change in light absorbance or intensity of scattered light.

### Background of the Invention

In immunological diagnosis, one of the three methods - RIA (radio immunoassay), EIA (enzyme immunoassay) and latex agglutination reaction - is generally employed. Among these, immunological diagnosis utilizing latex agglutination reaction is advantageous from the viewpoint of safety and speed of measurement. (Refer to Japanese Patent Publication No. 58-11575('83).)

The generally employed latex agglutination reaction comprises sensitizing a latex having a certain average particle size with an antibody against the antigen to be assayed or an antigen against the antibody to be assayed and reacting the sensitized latex with urine, blood serum or blood plasma of patients in an aqueous medium in a cell of plastic or glass. If the object substance is present in the cell, the latex agglutinates. The reaction mixture is irradiated from outside of the cell with light having a suitable wavelength selected from the range of 0.3 - 2.4 µm, change in absorbance or intensity of the scattered light is measured and thus the antigen or antibody can be assayed. Practically, a calibration curve representing the relation between concentration of the antigen or antibody and average rate of reaction or the relation between concentration thereof and change in absorbance, etc. is prepared beforehand by using known samples of different concentration levels, and the measured change in absorbance or reaction rate of a sample to be assayed is compared with the calibration curve, and thus the concentration can be determined.

Recently, however, there has risen in immunological diagnosis a strong need to assay to the order of ng/mℓ trace cancer marker substances in urine, blood serum, blood plasma, etc. such as α-fetoprotein (AFP), carcinoembryoic antigen (CEA), etc. The concentration range in which assay must be performed differs from substance to substance and samples in the concentration range of the order of from ng/mℓ to the order of µg/mℓ must be assayed. When high sensitivity measurement is carried out using latex agglutinating reaction reagents, it is advantageous:

    (a) To use a latex having a relatively large average particle size;

    (b) To sensitize the latex with a larger amount of antibody or antigen;

    (c) To use an antibody or an antigen having high specificity and affinity to the corresponding antigen or antibody; and

    (d) To employ sensitized latex of a concentration as high as possible within the range in which measurement of absorbance or intensity of scattered light is possible.

To carry out the above (b), (c) or (d) increases reagent cost since a larger amount of expensive antibody or antigen is used. Even so, however, the effect is not entirely satisfactory. Some difficulty is encountered in employing measure (c). As to AFP for instance, with respect to the above (a), measurement must be carried out in a concentration range of from several ng/mℓ to 500 ng/mℓ. When a latex of an average particle size of 0.2 µm which has been sensitized with AFP antibody is used, the measurable concentration range is 25 ng/mℓ ~ 2000 ng/mℓ. But when a latex of an average particle size of 0.5 µm is used, the measurable concentration range is 10 ng/mℓ ~ 200 ng/mℓ. That is, increase in sensitivity is achieved in lower concentrations, but in the higher concentration range, the calibration curve loses its linearity and the high end of the measurable range is lowered to 200 ng/mℓ.

### Disclosure of the Invention

The present invention is intended to solve the above described problems, and to provide a method of measurement of antigen-antibody reactions based on latex agglutination, which enables not only assay of substances of low concentrations but also assay of substances of high concentrations to some extent without additional procedures of dilution.

That is to say, the present invention makes possible assay of antigen or antibody in a wide range of from low concentration to high concentration by employing a mixture of two or more kinds of latexes of different average particle sizes. The gist thereof is a method of measuring antigen-antibody reactions characterized by reacting a suspension prepared by sensitizing two or more kinds of latexes of different average particle size with an antibody or an antigen and thereafter mixing them or a suspension prepared by mixing two or more kinds of latexes of different average particle sizes and thereafter sensitizing the mixture with an antibody or an antigen with an antigen against said antibody with which the latexes have been sensitized or an antibody against said antigen with which the latexes have been sensitized, in an aqueous medium, irradiating the reaction mixture with light having a wavelength of 0.3 ~ 2.4 µm and measuring change in absorbance or intensity of scattered light.

In the method of the present invention, the preparation of a latex reagent which comprises sensitizing two, three or more kinds of latexes of different average particle sizes respectively with an antibody or an antigen and mixing the sensitized latexes in a predetermined ratio to furnish a reagent which is provided with both a wide latitude in measurable range achieved by smaller particle latexes and high sensitivity in the lower concentration range achieved by larger particle latexes. The same results are obtained when two, three or more kinds of latexes of distinctly different particle sizes are mixed in a predetermined ratio and thereafter sensitized with an antibody or an antigen.

In preferred embodiments of the present invention, one of the latexes is selected from latexes having average particle sizes of 0.05 ~ 0.3 μm and another is selected from latexes having average sizes of 0.3 ~ 1.0 μm.

More preferably, one latex is selected from those having average particle sizes of 0.1 ~ 0.3 μm and another latex is selected from those having average particle sizes of 0.4 ~ 0.8 μm.

Still more preferably, one latex is selected from those having average particle sizes 0.2 ~ 0.3 μm and another latex is selected from those having average particle size of 0.4 ~ 0.5 μm.

When two kinds of latexes are used, the two latexes are preferably mixed in a ratio of 1:10 to 10:1, more preferably in a ratio of 1:5 to 5:1 and still more preferably in a ratio of 1:3 to 3:1.

The method of this invention will now be described in detail.

The measurement of change in absorbance or intensity of scattered light for assaying antigen or antibody in the latex agglutination reaction employed in the present invention can be carried out in accordance with the method disclosed in Japanese Patent Publication No. 58-11575('83), Japenese Laid-Open Patent Publication No. 54-109494('79), etc.

That is, the amount (or concentration) of antigen or antibody in a sample can be measured by using insoluble carrier particles preferably having a particle size of 1.6 μ or less, sensitizing them with an antibody or an antigen, reacting them with the corresponding antigen or antibody in a sample, and measuring absorbance of the reaction mixture with light of a wavelength preferably in the range of 0.6 to 2.4 μ. The employed light should preferably be of a wavelength of at least 1.5 times the average diameter of the carrier particles used, more preferably at least twice, and most preferably at least 2.5 times said diameter.

As carrier particles, preferred are fine particles of organic high polymers having the above-mentioned average particles sizes which are substantially insoluble in the liquid media in which assay is carried out, such as latexes of polystyrene, styrene-butadiene copolymers, etc., which are obtained by emulsion polymerization.

Such insoluble carrier particles, latex particles for instance, are sensitized with an antibody or an antigen which is reactable with the antigen or the antibody to be assayed in a sample. The sensitization can be effected by physical adsorption or chemical adsorption of antibody molecules or antigen molecules on the carrier particles.

In order to assay an antigen or an antibody in a sample which contains an unknown amount of the antigen or the antibody, a standard sample which contains a known amount of the antigen or the antibody is prepared, this standard sample is diluted to different concentrations to provide a plurality of diluted standard samples, these samples are reacted with insoluble carrier particles sensitized with a predetermined amount of the corresponding antibody or antigen under predetermined conditions, absorbances of the reaction mixtures are measured, and a calibration curve is plotted representing the relation between the amount (concentration) of the antigen or antibody and the absorbance. Then a sample is reacted with the same sensitized carrier as that used for the plotting of the calibration curve under the same conditions as employed in the plotting of the curve, the absorbance of the reaction mixture is measured, the obtained absorbance is collated with the calibration curve, and thus the amount (concentration) of the antigen or antibody in the sample can be determined.

Alternatively, a calibration curve can be plotted with respect to the relation between the amounts of an antigen or antibody in standard samples and the reaction time until a predetermined absorbance is reached or the time-course change (reaction rate) of absorbance. Then, the sensitized particles are reacted with a sample, and the reaction time until a predetermined absorbance value is reached or the reaction rate is measured. Thus, the amount (concentration) of the antigen or antibody in the sample can be determined.

Measurement of scattered light is performed by having insoluble carrier particles of an average particle size of not more than 1.6 μ carry an antibody or an antigen, reacting the particles carrying the antibody or the antigen with the antigen or the antibody of said antibody or said antigen in a liquid medium, irradiating the reaction mixture with light preferably having a wavelength selected from the range of from 0.8 to 2.4 μ at two or more points of time after the start of the reaction and measuring the intensity of the light scattered by the reaction mixture.

An antigen-antibody reaction can be conducted by either of the following two methods:

(a) a suspension of carrier particles carrying a particular antibody or antigen is reacted with a sample solution containing the object antigen or antibody as is or diluted or concentrated; or

(b) a sample solution containing antibody or antigen to be assayed is first reacted with a predetermined known amount of its antigen or antibody in order to reduce the amount of the object antibody or antigen and then the reaction mixture is reacted with a suspension of insoluble particles carrying the corresponding antigen or antibody in order to assay the remaining antibody or antigen.

After the start of the reaction, at two or more points of time, the reaction mixture is irradiated with light of a particular wavelength, and the light scattered by the antigen-antibody reaction mixture is measured. Specific procedures therefor are:

(A) A standard sample containing a predetermined amount of an antigen or antibody is prepared, and it is diluted to different concentrations to provide diluted standard samples. These diluted samples are reacted with insoluble particles carrying a predetermined amount of a particular antibody or antigen under predetermined conditions, for instance at room temperature for several minutes to two hours, the intensity of the light scattered by this reaction mixture is measured and a calibration curve is prepared representing the relation between the amount (concentration) of the antigen or antibody and the intensity of scattered light. Then a sample and the same sensitized carrier as used for the preparation of the calibration curve are reacted under substantially the same conditions and the scattered light is measured. The amount (or concentration) of the antigen or antibody can be determined by comparing and collating the intensity of the scattered light with said calibration curve.

(B) A calibration curve representing the relation between the reaction time until the intensity of scattered light reaches a pre-determined level (several seconds to ten minutes at room temperature, for instance) and the amount (concentration) of the antigen or antibody in the standard sample used is prepared. A sample is reacted with said sensitized carrier under substantially identical conditions, and the reaction time until the intensity of scattered light reaches a predetermined level is measured. Thus the amount (concentration) of the antigen or antibody in the sample can be determined.

(C) A standard sample containing a predetermined amount of an antigen or antibody is prepared, and this sample is diluted to different concentrations to prepare a plurality of diluted standard samples. They are reacted insoluble particles sensitized with a predetermined amount of a corresponding antibody or antigen under the same condition, and the scattered light is measured at two or more points of time a short time (at least 2 ~ 3 seconds, preferably not less than about 5 seconds, for instance) after the start of the reaction, i.e. after the reaction has been stabilized and the intensity of the scattered light has begun to increase stationarily. Thus a calibration curve is prepared which represents the relation between the amount (concentration) of said antigen or antibody and the increase rate of the intensity of the scattered light per unit time. A sample to be assayed is reacted with the sensitized particles under substantially identical conditions as when the calibration curve has been prepared, and the increase rate per unit time in the intensity of the scattered light is determined. The amount (concentration) of the antigen or antibody in the sample can be determined by comparing and collating the measured increase rate of the scattered light intensity with the calibration curve. The measurement of the scattered light can be easily carried out using an integrating sphere used for measurement of absorption spectrum of ordinary emulsion samples, said integrating sphere having an inside surface blackened at the position where the incidental light transmitting the sample cell impinges, or alternately using an ordinary scattered light measuring apparatus generally employed. In the present invention, it is necessary to use two or more latexes having different average particle sizes.

The average sizes are usually selected from the range of from about 0.05 to 1.0 μm. The difference in the average particle size is usually not less than 0.1 μm, preferably not less than 0.2 μm, although it differs depending upon (i) the lowest measurable concentration (sensitivity),(ii) the concentration range (usually up to about 1000 times the lowest measurement concentration), (iii) the latex concentration, (iv) the antigen-antibody reactivity, etc. It is preferred to select the average size of one latex from the range of 0.05 ~ 0.3 μm and that of the other from the range of 0.3 ~ 1 μm when two latexes are used.

When two latexes are used, the mixing ratio (volume ratio) of the latexes is 1:10 ~ 10:1, preferably 1:5 ~ 5:1, more preferably 1:3 ~ 3:1. The intended lowest measurable concentration (sensitivity) differs depending upon the species of antigen and antibody. For instance, it is 5 ~ 10 ng/mℓ for AFP and around 10 ng for ferritin.

The calibration curve is usually plotted to about 1000 times the lowest measurable concentration.

Brief Explanation of the Drawing

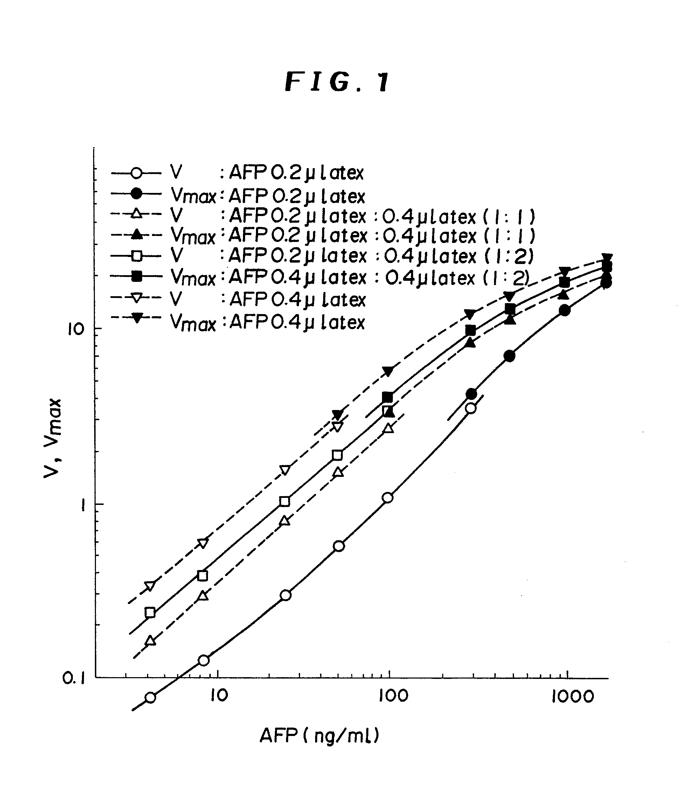

Fig. 1 is a graph showing calibration curves representing the relation between the average reaction rate or the maximum reaction rate and the concentration of AFP to be assayed when anti-AFP latex is used in the method of this invention.

Detailed Description of Embodiments

Now the invention will be more specifically explained. In the assay of AFP as illustrated later in Example 1 for instance, a polystyrene latex having an average particle size of 0.2 μm and a polystyrene latex having an average particle size of 0.4 μm are respectively sensitized with AFP antibody (anti-AFP specific antibody obtained by immunizing an animal with human AFP), and are mixed in ratios of 1:1 and 1:2 (volume). The obtained calibration curves which represent the relation between the AFP concentration and the average reaction rate V or the maximum reaction rate $V_{max}$ (the maximum value of reaction rates measured at 50 points of time at 12 second intervals) are shown in Fig. 1. As seen in the figure, the inclination of the calibration curves becomes steeper in the range of 100 ng/mℓ ~ 2000 ng/mℓ, as the proportion of the particles having the average particle size of 0.2 μm increases. As shown in Table 3, this means that when standard samples of known antigen or antibody concentration are assayed using respective calibration curves, a latex reagent containing a higher ratio of 0.2 μm particles has a higher upper limit in terms of measurable range. The measurable upper concentration limit for the respective latexes is observed as follows. For 0.2 μ anti-AFP latex (latex sensitized with anti-AFP antibody), coefficient of variation (C.V.) is as low as 1.77% and the measurable upper limit is 2,000 ng/mgℓ when the antigen or antibody concentration in the standard sample is 2,000 ng/mℓ. For 0.4 μm anti-AFP latex, C.V. is 8.26% at 300 ng/mℓ and the upper limit is as low as 200 ng/mℓ. On the other hand, for an anti-AFP latex comprising a 1:1 mixture of a 0.2 μ latex and a 0.4 μ latex, C.V. is 4.76% at 500 ng/mℓ of concentration; for an anti-AFP latex comprising a 1:2 mixture of said latexes, C.V. is 5.46% at 500 ng/mℓ of concentration. The measurable upper limit concentration is 500 ng/mℓ for these two mixed anti-AFP latexes.

It can be said that the reactivity of a reagent is higher as the difference between the average reaction rate V of the blank test sample and the average reaction rate V at the standard sample concentration of 4.2 ng/mℓ as indicated in Table 1 is greater than the standard deviation S.D. of the average reaction rate V of the blank test sample. As can be seen from Table 1, as the content ratio of the latex having the average particle size of 0.4 μ increases, the average reaction rate at lower concentration increases. In the data on the standard samples in Table 3, the lower limit of the measurable concentration range for the 0.2 μ anti-AFP latex is 25 ng/mℓ in view of C.V. being not more than 10%, and that for the 0.4 μ anti-AFP latex is 4.2 ng/mℓ. The coefficient of variation (C.V.) of the anti-AFP latexes respectively comprising a 1:1 and 1:2 mixture of 0.2 μ latex and a 0.4 μ latex are respectively 7.31% and 6.37%, and the measurable lower limit concentration is 4.2 ng/mℓ for both. The measurable concentration range of the 0.2 μ anti-AFP latex is 25 ng/mℓ ~ 2,000 ng/mℓ, that is, it is adequate on the high concentration side, but inadequate on the low concentration side, while that of the 0.4 μ anti-AFP latex is 5 ng/mℓ ~ 200 ng/mℓ, that is, this reagent is unsatisfactory in that the measurable range is limited on the high concentration side. On the other hand, the measurable concentration range of both 1:1 and 1:2 mixed anti-AFP latexes of a 0.2 μ latex and 0.4 μ latex is 5 ng/mℓ ~ 500 ng/mℓ, satisfactory on both the upper side and lower sides in view of current clinical test levels.

Example 2 relates to an anti-ferritin latex. Table 4 shows data for calibration curves of a 0.3 μ and 0.5 μ mixed anti-ferritin latex and data for calibration curves of a 0.5 μ anti-ferritin latex. Like in the case of the anti-AFP latex, when small particle size latexes are mixed, the average reaction rate V becomes lower on the low concentration side. When the difference between $V_{max}$ at 300 ng/mℓ and $V_{max}$ at 400 ng/mℓ is compared, the inclination of the calibration curve for the 0.3 μ and 0.5 μ mixed anti-ferritin mixture is greater, and it is apparent that this reagent is suitable for measurement in the higher concentration range. Table 5 shows measurement data of low concentration standard samples of a 0.3 μ anti-ferritin latex, 3:1 and 1:1 mixed anti-ferritin latexes of 0.3 μ average particle size and 0.5 μ average particle size, and a 0.5 μ anti-ferritin latex. It can be seen that the average reaction rate V increases as the content ratio of the 0.5 μ latex increases and the 0.5 μ anti-ferritin latex is superior ragarding measurement in the low concentration range. From the above-described results, it can be said that in the reagent prepared by mixing sensitized latexes having different average particle sizes, properties of the component latexes are reflected in accordance with the mixing ratio of the components. That is to say, it is possible to obtain a reagent having a measurement range suitable for the substance to be assayed and thus having a higher degree of freedom by varying the mixing ratio of sensitized latexes of different particle sizes.

Now the invention will be illustrated by way of working examples. However, the invention is not limited to the following examples only.

Example 1

(Preparation of Reagent)

A latex containing 1% (w/v) of polyethylene particles of average particle size of 0.2 μ was sensitized by thoroughly mixing it in a 0.1 M/ℓ Tris-HCℓ buffer solution (pH 8.0 ~ 8.4) with an anti-AFP antibody obtained by immunizing an animal with human AFP. The mixture was centrifuged and the supernatant was removed. The precipitated latex particles were redispersed with a 0.1 M/ℓ Tirs-HCℓ buffer solution containing 0.3% bovine serum albumin (BSA) (pH 8.0 ~ 8.4) and thus a 0.2 μ anti-AFP latex was obtained. By the same procedure, a 0.4 μ anti-AFP latex was obtained, which was mixed with the 0.2 μ anti-AFP latex prepared as described above in volume ratios of 1:1 and 1:2 and thoroughly stirred. Thus mixed anti-AFP latex reagents were obtained.

(Preparation of Calibration Curves)

The mixed anti-AFP latex reagents were mixed with standard samples of known antigen or antibody concentrations (0 ng/mℓ, 4.2 ng/mℓ, 8.3 ng/mℓ, 25 ng/mℓ, 50 ng/mℓ, 100 ng/mℓ, 300 ng/mℓ, 500 ng/mℓ and 1000 ng/mℓ and 2000 ng/mℓ) and the reaction rates were measured by an "LPIA®100" system (manufactured by Mitsubishi Chemical Industries Limited). In this system, 10 μℓ of a sample was taken and was mixed with 50 μℓ of a 0.1 M/ℓ Tris-HCℓ buffer solution containing 0.05% BSA and 0.9% NaCℓ (pH 8.0 ~ 8.4), and the thus treated sample was further mixed with 200 μℓ of a 0.2 M/ℓ Tris-HCℓ buffer solution containing 0.25% BSA and 0.9% NaCℓ (pH 8.0 ~ 8.4) and 40 μℓ of a latex dispersion and thoroughly mixed for reaction in a plastic cell. The light absorption due to the latex agglutination caused by the antigen-antibody reaction was measured at a wave length of 950 nm and the change in light absorbance was calculated with average reaction rate V and maximum reaction rate $V_{max}$.

Table 1 shows data of average reaction rate for calibration curves obtained with a 0.2 μ anti-AFP latex, 1:1 and 1:2 mixed anti-AFP latexes of 0.2 μ particles and 0.4 μ particles, and a 0.4 μ anti-AFP latex.

Table 2 shows data of maximum reaction rates for calibration curves obtained with the above-mentioned four anti-AFP latexes.

Table 3 shows data measured with the four anti-AFP latexes using the standard samples as samples to be assayed. For the 0.2 μ anti-AFP latex, a calibration curve was plotted with the average reaction rate V in the range of not more than 300 ng/mℓ and with the maximum reaction rate $V_{max}$ in the range of not less than 500 ng/mℓ. For the 1:1 and 1:2 mixed anti-AFP latexes of 0.2 μ particles and 0.4 μ particles, calibration curves were plotted with the average reaction rate V in the range of not more than 100 ng/mℓ and with the maximum reaction rate $V_{max}$ in the range of not less than 300 ng/mℓ. For the 0.4 μ anti-AFP latex, calibration curves were plotted with the average reaction rate V in the range of not more than 50 ng/mℓ and with the miximum reaction rate $V_{max}$ in the range not less than 100 ng/mℓ. These calibration curves are shown in Fig. 1.

Table 1    Data for calibration curves for latexes sensitized
with anti-AFP specific antibody

(Relation between concentration of standard samples and reaction rate V)

| Conc. / Species of latex | 0 ng | 4.2 ng | 8.3 ng | 25 ng | 50 ng | 100 ng | 300 ng | 500 ng | 1000 ng | 2000 ng |
|---|---|---|---|---|---|---|---|---|---|---|
| 0.2 $\mu$ anti-AFP latex | 0.77 | 2.00 | 3.32 | 7.66 | 14.84 | 29.35 | 104.27 | 180.79 | 274.28 | 270.91 |
| 0.2 $\mu$:0.4 $\mu$ (1:1) anti-AFP latex | 0.45 | 4.21 | 7.51 | 21.00 | 40.98 | 77.06 | 185.97 | 224.08 | 227.99 | 208.43 |
| 0.2 $\mu$:0.4 $\mu$ (1:2) anti-AFP latex | 0.73 | 6.05 | 9.85 | 27.64 | 52.70 | 97.14 | 206.56 | 227.88 | 232.84 | 206.68 |
| 0.4 $\mu$ anti-AFP latex | 1.21 | 8.89 | 15.60 | 42.67 | 80.38 | 140.51 | 209.79 | 204.64 | 180.92 | 153.37 |

Table 2    Data for calibration curves for latexes sensitized
with anti-AFP specific antibody

(Relation between concentration of standard samples and maximum reaction rate $V_{max}$)

| Conc. / Species of latex | 0 ng | 4.2 ng | 8.3 ng | 25 ng | 50 ng | 100 ng | 300 ng | 500 ng | 1000 ng | 2000 ng |
|---|---|---|---|---|---|---|---|---|---|---|
| 0.2 $\mu$ anti-AFP latex | 21.48 | 18.88 | 18.88 | 29.30 | 30.60 | 51.43 | 134.77 | 215.49 | 416.67 | 662.76 |
| 0.2 $\mu$:0.4 $\mu$ (1:1) anti-AFP latex | 18.23 | 18.23 | 18.88 | 37.76 | 64.45 | 107.42 | 263.02 | 361.98 | 498.05 | 684.24 |
| 0.2 $\mu$:0.4 $\mu$ (1:2) anti-AFP latex | 16.93 | 20.83 | 29.95 | 47.53 | 74.87 | 123.05 | 301.43 | 389.32 | 572.92 | 745.44 |
| 0.4 $\mu$ anti-AFP latex | 13.67 | 23.44 | 28.65 | 65.76 | 102.86 | 173.83 | 372.40 | 482.42 | 623.05 | 733.07 |

0 263 731

Table 3    Measured concentrations of standard samples

n=10

| Species of latex | Conc. | 0 ng/ml | 4.2 ng/ml | 8.3 ng/ml | 25 ng/ml | 50 ng/ml | 100 ng/ml | 300 ng/ml | 500 ng/ml | 1000 ng/ml | 2000 ng/ml |
|---|---|---|---|---|---|---|---|---|---|---|---|
| 0.2 μ anti-AFP latex | mean | 0.83 | 4.15 | 8.54 | 24.33 | 50.59 | 100.14 | 300.53 | 494.56 | 1011.66 | 1990.65 |
| | S.D. | 0.91 | 0.53 | 1.22 | 0.70 | 0.79 | 0.60 | 4.34 | 1.62 | 19.06 | 35.28 |
| | C.V. % | 103.68 | 12.88 | 14.31 | 2.88 | 1.56 | 0.60 | 1.44 | 0.33 | 1.88 | 1.77 |
| 0.2 μ:0.4 μ (1:1) anti-AFP latex | mean | 0.36 | 4.26 | 8.09 | 24.99 | 51.31 | 99.23 | 309.99 | 506.61 | 918.97 | 2124.64 |
| | S.D. | 0.19 | 0.31 | 0.68 | 0.15 | 1.27 | 1.32 | 3.05 | 24.10 | 50.40 | 168.20 |
| | C.V. % | 51.11 | 7.31 | 8.42 | 0.59 | 2.48 | 1.34 | 0.98 | 4.76 | 5.48 | 7.92 |
| 0.2 μ:0.4 μ (1:2) anti-AFP | mean | 0.32 | 4.36 | 7.76 | 25.49 | 51.85 | 99.40 | 312.43 | 512.48 | 990.69 | 2038.04 |
| | S.D. | 0.01 | 0.28 | 0.39 | 0.33 | 0.13 | 3.19 | 0.91 | 28.0 | 37.79 | 135.38 |
| | C.V. % | 3.03 | 6.37 | 5.07 | 1.31 | 0.26 | 3.21 | 0.29 | 5.46 | 3.81 | 6.64 |
| 0.4 μ anti-AFP latex | mean | 0.38 | 4.23 | 8.17 | 25.40 | 49.98 | 100.04 | 301.19 | 494.52 | 1023.95 | 1978.07 |
| | S.D. | 0.14 | 0.16 | 0.24 | 0.81 | 2.02 | 0.20 | 24.88 | 42.88 | 84.72 | 155.07 |
| | C.V. % | 38.40 | 3.82 | 2.93 | 3.17 | 4.05 | 0.20 | 8.67 | 8.67 | 8.27 | 12.75 |

$$C.V. (\%) = \frac{S.D.}{mean} \times 100\%$$

8

0 263 731

Example 2

(Preparation of Reagent)

A latex containing 1% (w/v) of polystyrene particles of an average particle size of 0.3 μ was sensitized by thoroughly mixing it in the same buffer solution as used in Example 1 with an anti-ferritin specific antibody obtained by immunizing an animal with human ferritin. The mixture was centrifuged and the supernatant was removed. The precipitated latex particles were redispersed in the same manner as in Example 1 and 0.3 μ anti-ferritin latex was obtained. By the same procedure, a 0.5 μ anti-ferritin latex was prepared, which was mixed with the 0.3 μ anti-ferritin latex prepared as described above in volume ratios of 1:1 and 1:2 and thoroughly stirred. Thus mixed anti-ferritin latex reagents were obtained.

(Preparation of Calibration Curves)

The mixed anti-ferritin latex reagents were mixed with standard samples of known antigen or antibody concentrations (0 ng/mℓ, 6.3 ng/mℓ, 12.5 ng/mℓ, 25 ng/mℓ, 50 ng/mℓ, 100 ng/mℓ, 300 ng/mℓ and 400 ng/mℓ) and the reaction rates were measured by the LPIA® 100" system in the same manner as in Example 1.

Table 4 shows data for calibration curves obtained with a 1:1 mixed anti-ferritin latex of 0.3 μ particles and 0.5 μ particles and a 0.5 μ anti-ferritin latex. Calibration curves were plotted with the average reaction rate V in the range of not more than 100 ng/mℓ and with the maximum reaction rate $V_{max}$ in the range of not less than 200 ng/mℓ. Table 5 shows values of the average reaction rate V in the low concentration range with respect to standard samples of a 0.3 μ anti-ferritin latex, 3:1 and 1:1 mixed anti-ferritin latexes of 0.3 μ particles and 0.5 μ particles and a 0.5 μ anti-ferritin latex.

Table 4    Data for calibration curves for latexes sensitized with anti-ferritin specific antibody

| | V | | | | | | $V_{max}$ | | |
|---|---|---|---|---|---|---|---|---|---|
| | 0 ng/mℓ | 6.3 ng/mℓ | 12.5 ng/mℓ | 25.0 ng/mℓ | 50 ng/mℓ | 100 ng/mℓ | 200 ng/mℓ | 300 ng/mℓ | 400 ng/mℓ |
| 0.3 µ :0.5 µ (1:1) anti-ferritin latex | 1.61 | 5.7 | 8.9 | 15.9 | 28.9 | 57.0 | 334.0 | 457.0 | 548.8 |
| 0.5 µ anti-ferritin latex | 2.03 | 7.5 | 12.9 | 22.5 | 44.5 | 81.0 | 360.0 | 513.0 | 570.3 |

Table 5    Data on latexes sensitized with antiferritin specific antibody in low concentration range

(Average reaction rate of standard samples in low concentration)

| Conc. of standard samples / Specific of latex | 0 | 3.2 ng/mℓ | 6.3 ng/mℓ | 12.5 ng/mℓ |
|---|---|---|---|---|
| 0.3 µ anti-ferritin latex | 1.44 | 2.92 | 3.27 | 5.22 |
| 0.3 µ :0.5 µ (3:1) anti-ferritin latex | 1.79 | 2.45 | 3.67 | 6.54 |
| 0.3 µ:0.5 µ (1:1) anti-ferritin latex | 1.61 | 4.88 | 5.7 | 8.9 |
| 0.5 µ anti-ferritin latex | 2.03 | 3.8 | 7.5 | 13.5 |

# 0 263 731

## Claims

1. A method for measuring antigen-antibody reactions comprising reacting a suspension prepared by sensitizing two or more kinds of latexes having different average particle sizes with an antibody or antigen and mixing them or a suspension prepared by mixing two or more kinds of latexes having different average particle sizes and thereafter sensitizing the mixture with an antibody or an antigen with an antigen against the antibody with which the latexes have been sensitized or an antibody against the antigen with which the latexes have been sensitized in an aqueous medium; irradiating the reaction system with light having a wave length of $0.3 \sim 2.4\ \mu m$; and measuring change in light absorbance or intensity of scattered light, and thus measuring the antigen-antibody reaction.

2. The method for measuring antigen-antibody reactions as set forth in Claim 1, wherein a latex having an average particle size in the range of $0.05 \sim 0.3\ \mu m$ and another latex having an average particle size in the range of $0.3 \sim 1.0\ \mu m$ are mixed.

3. The method for measuring antigen-antibody reactions as set forth in Claim 2, wherein a latex having an average particle size in the range of $0.1 \sim 0.3\ \mu m$ and another latex having an average particle size in the range of $0.4 \sim 0.8\ \mu m$.

4. The method for measuring antigen-antibody reactions as set forth in Claim 3, wherein a latex having an average particle size in the range of $0.2 \sim 0.3\ \mu m$ and another latex having an average particle size in the range of $0.4 \sim 0.5\ \mu m$.

5. The method for measuring antigen-antibody reactions as set forth in Claim 1, wherein two latexes having different average particle sizes are mixed in a ratio of 1:10 to 10:1.

6. The method for measuring antigen-antibody reactions as set forth in Claim 5, wherein two latexes having different average particle sizes are mixed in a ratio of 1:5 to 5:1.

7. The method for measuring antigen-antibody reactions as set forth in Claim 6, wherein two latexes having different average particle size are mixed in a ratio of 1:3 to 3:1.

11

# FIG. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 019 741 (BEHRINGWERKE AG) * Page 2, line 28 - page 3, line 21; page 8, line 1 - page 9, line 17 * | 1-7 | G 01 N 33/546 |
| X | EP-A-0 126 450 (I. TRIPATZIS) * Abstract, lines 7-10; claims; page 2, lines 9-15 * | 1 | |
| X | FR-A-2 319 131 (COULTER ELECTRONICS INC.) * Page 8, lines 2-9; page 11, lines 2-25 * | 1 | |
| X | US-A-4 184 849 (C.L. CAMBIASO et al.) * Column 3, lines 17-22; column 4, lines 12-17; column 8, lines 1-3 * | 1 | |

TECHNICAL FIELDS SEARCHED (Int. Cl.4)

G 01 N 33/00

The present search report has been drawn up for all claims

| Place of search | Date of completion of the search | Examiner |
|---|---|---|
| THE HAGUE | 04-01-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)